# EUROPEAN PATENT APPLICATION

(11) **EP 0 578 481 A2**
(43) Date of publication of application: **12.01.1994**
(21) Application number: 93305309.2
(22) Date of filing: 06.07.1993
(51) Int. Cl.: C11D 9/22, C11D 3/37, A61K 7/48

(54) **Cleansing bar to condition skin and/or hair**

(30) Priority: 08.07.1992 US 910371
(71) Applicant: Colgate-Palmolive Company, New York, N.Y. 10022-7499 (US)
(72) Inventor: Patel, Amrit, Dayton, New Jersey (US); Robbins, Clarence R., Martinsville, New Jersey (US); Hilliard, Peter R., Jr., Far Hills, New Jersey (US)
(74) Representative: Kearney, Kevin David Nicholas

(57) **Abstract**

A solid soap composition with from about 94% to about 99% by weight of a soap base and from about 6% to about 1% by weight of a water-insoluble silicone having a viscosity of from about 40,000 to 120,000 cP cleans and conditions hair and skin to provide softer, smoother hair and skin. The composition may be in the form of a soap bar.

## Description

### BACKGROUND OF THE INVENTION

### Field Of The Invention:

This invention relates to a solid soap composition which can be shaped or formed.into a bar, cake or other solid product useful both as a shampoo for the hair and scalp and as a soap for the skin or body. More particularly, the present invention relates to a solid soap composition that reduces cracking and flaking of skin relative to soap alone, and which decreases soap damage to the skin.

### Description Of The Prior Art

Soaps have been employed for many years as cleansing agents for the skin and other substrates. In more recent years, it has become the practice to improve the properties of the soap by incorporation of various additives. For example, coconut oil or palm kernel oil has been added to improve lather formation. So-called "super fatting" agents such as lanolin, fatty acid derivatives and lecithin have also been added to prevent the degreasing effect of the soap on human skin.

British Patent No. 998,076 discloses the incorporation of silicone liquids, such as polydimethylsiloxanes, polyphenylmethylsiloxanes and polysiloxane-polyoxyalkylene copolymers, into shaving creams to lubricate the skin.

U.S. Patent No. 4,279,765 discloses a soap composition which contains from 0.01 to 6% by weight of a polydiorganosiloxane having a molecular weight of at least 2000 and in which at least one of the silicon-bonded substituents is a monovalent group composed of carbon, hydrogen, nitrogen and, optionally, oxygen, the monovalent group having therein at least one amino group.

U.S. Patent No. 4,919,838 discloses a bar composition which is used both as a shampoo for hair or scalp and as a cleansing soap for the skin or body. The bar composition may contain a hair conditioning agent, such as a silicone glycol copolymer (Dimethicone Copolyol), in an amount of up to 0.75% by weight.

German Democratic Republic Patent No. 76293 discloses cleansing bars and pastes containing a methylsilicone oil to protect the skin from aggressive substances and to improve processability. The examples shown only teach the protective effects of the 200 centistoke (cSt) methylsilicone oil.

Various types of silicon-containing polymers have been used in shampoos, as described in U.S. Patent Nos. 4,559,227, 4,741,855 and 4,842,850. These shampoos are of the liquid type, including gels or pastes.

### SUMMARY OF THE INVENTION

An object of the present invention is to provide a solid soap composition for cleaning and conditioning hair and skin to provide softer, smoother hair and skin; and also to reduce the cracking and flaking of skin relative to the use of soap alone, and to improve the moisture retention of skin after treatment with soap. An object of the present invention is also to provide a solid soap composition wherein a liquid component contained within the bar will not bleed out of the bar. These goals are achieved by the provision of a solid soap composition for cleaning and conditioning hair and skin to provide softer, smoother hair and skin, comprising: from about 93% to about 99.5% by weight of a soap base; and from about 7% to about 0.5% by weight of a water-insoluble silicone. The water-insoluble silicone should have a number average molecular weight range from about 15,000, with a polydispersity of less than or equal to about 3.8, to about 35,000, with a polydispersity of less than or equal to about 4.5. The viscosities of the water-insoluble silicones should range from about 20,000 centipoise to 200,000 centipoise, and preferably from 25,000 cP to 150,000 cP.

### BRIEF DESCRIPTION OF THE DRAWINGS

Fig. 1 is a graph illustrating the effect of a water-insoluble silicone on the acute surfactant induced damage to the water barrier of excised pig skin, as measured by transepidermal water loss (TEWL), produced by a super fatted soap base (60T/40C). (60% tallow/40% coco soap)

Fig. 2 is a graph illustrating the effect of a water-insoluble silicone on the acute surfactant induced damage to the water barrier of excised pig skin, as measured by transepidermal water loss (TEWL), produced by a conventional soap base (70T/30PKO) (70% tallow/30% palm kernel oil soap)

Fig. 3 is a graph illustrating the effect of the viscosity of a water-insoluble silicone on in vitro moisture retention of skin, as measured by % area of dermal hydration.

Fig. 4 is a graph illustrating the effect of the viscosity of a water-insoluble silicone on the surface deposition of silicone on skin (given as Si/N (atomie ratio of silicon and nitrogen) ratio, as measured by ESCA, (electron spectroscopy for chemical analysis).

Fig. 5 is a graph illustrating the effect of the viscosity of a water-insoluble silicone on skin moisturization, as measured by epidermal conductance.

Fig. 6 is a graph illustrating the effect of the viscosity of a water-insoluble silicone on skin water barrier damage, *in vitro,* as measured by normalized transepidermal water loss (TEWL).

Fig. 7 is a graph illustrating the effect of concentration of a water-insoluble silicone on skin smoothness, as measured by skin friction (reduction in static friction).

Fig. 8 is a graph illustrating the effect of a water-insoluble silicone on the soap-induced cracking of skin.

Fig. 9 is a graph illustrating the effect of a water-insoluble silicone on the soap-induced flaking of skin.

### DETAILED DESCRIPTION OF THE INVENTION

The object of the present invention is to condition skin and/or hair from a bar cleansing product. In order to achieve this goal the present invention provides a solid soap composition comprising from about 93% to about 99.5% by weight, preferably 96% to 98% by weight, of a soap base and from about 7% to about 0.5% by weight, preferably about 4% to 2% by weight, of a water-insoluble silicone having a viscosity of from about 20,000 to about 200,000 cP.

The soap base may be of natural or synthetic origin or a mixture of soap base and a synthetic base, or synthetic detergent soap base (syndet).

By a natural soap base is meant a salt of a fatty acid which is obtained by saponifying animal or vegetable fats or oils with alkali, such as sodium or potassium hydroxide, or alkanolamines, such as ethanolamine, or the like; or mixtures of such salts. It is generally preferred that the natural soap base be primarily saponified tallow, i.e., a major portion of tallow soap and a minor portion of an additional natural soap. Preferably, the tallow soap comprises 60-70% by weight of the natural soap base. More preferably, the natural soap base is a 60/40 mixture of tallow soap (T) and coconut oil (coco) (C) soap or a 70/30 mixture of tallow soap and palm kernel oil (PKO) soap.

By a synthetic detergent soap base, it is meant an anionic, amphoteric, or nonionic synthetic detergent composition. Suitable anionic synthetic detergents include alkyl benzene sulfonates, alkyl sulfates, alkyl benzene polyoxyethylene sulfonates, sulfated monoglycerides, alcohol ether sulfates and the like. Suitable nonionic synthetic detergent compositions include those produced by the condensation of alkylene oxides with organic hydrophobic compounds having an active hydrogen atom, such as those sold under the names Neodol®, Pluronic®, Igepal® or Tween®.

When the soap base comprises a mixture of a natural soap base and a synthetic detergent soap base, the synthetic detergent soap base may comprise up to 60% by weight of the total soap base, more preferably up to 50% by weight of the total soap base, and most preferably up to 30% by weight of the total soap base.

The water-insoluble silicones suitable for use in the present invention include the polydihydrocarbylsiloxanes of the formula 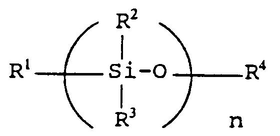
wherein R¹, R², R³ and R⁴ each independently represent a hydrocarbyl group and n is the degree of polymerization. Preferably, R² and R³ represent alkyl or aromatic groups; more preferably, R² and R³ represent lower alkyl groups (i.e., of 1-4 carbon atoms); and, most preferably, R²=R³=methyl. R¹ and R⁴ are preferably lower alkyl of 1 to 4 carbon atoms, and most preferably methyl. The degree of polymerization, n, is such that the polymer has a number average molecular weight in the range of from about 15,000, with a poly-dispersity of less than or equal to about 3.8, to about 35,000, with a polydispersity of less than or equal to about 4.5. The viscosities of these water-insoluble silicones should range from approximately 20,000 cP to 200,000 cP, and preferably from 25,000 cP to 150,000 cP, especially preferably from about 30,000 centipoise to 100,000 centipoise.

The solid soap composition of the present invention may be formulated by adding the water-insoluble silicone to chips of the soap base; and then mixing and distributing the silicone throughout the soap base by the normal soap mixing process of amalgamation, plodding, etc., and then pressing the solid soap composition into a desired shape, e.g., a bar.

Alternatively, the solid soap composition may be formulated by merely mixing the water-insoluble silicone plus an emulsifier with the chips of the soap base. The emulsifier is used in a silicone to emulsifier weight ratio of 1:5 to 5:1, preferably about 1:1. Suitable emulsifiers includes commercially available amides, such as cocodiethanolamide (CDEA) and Surfaclone LP-300, and ethoxylated alcohols and ethoxylated acids, such as Polysorbate 80.

Typical soap bar formulations according to this invention are set forth in the following Table.

**TABLE**

| INGREDIENT | % IN FORMULATION (BY WT.) | | |
|---|---|---|---|
| Soap Chips, e.g. 60/40 Coco/Tallow | 96 | 98 | 96 |
| Silicone | 4 | 2 | 2 |
| Emulsifier, e.g. CDEA | - | - | 2 |

The solid soap composition of the present invention may contain a variety of other conventional optional ingredients suitable for rendering such compositions more acceptable to the consumer. Such conventional optional ingredients are well-known to those of ordinary skill in the art and include, e.g., so-called "super-fatting" agents such as lanolin, fatty acid derivatives and lecithin; pearlescent aids such as ethyleneglycol distearate; preservatives such as benzyl alcohol, ethyl paraben, propyl paraben and imidazolidinyl urea; thickeners and viscosity modifiers such as a diethanolamide of a long chain fatty acid, i.e., cocomonoethanolamide, guar gum, methylcellulose, starch derivatives, fatty alcohols, sodium chloride, sodium sulfate, polyvinyl alcohol and ethyl alcohol; pH adjusting agents such as citric acid, sodium citrate, succinic acid, phosphoric acid, sodium hydroxide, sodium carbonate, etc.,; coloring agents such as any of the F,D&C and/or D&C dyes; whitening aids, such as titaniumdioxide; perfumes; sequestering agents such as tetrasodium ethylenediamine tetraacetate; and polymer plasticizing agents such as glycerin and propylglycol.

Such conventional optional ingredients are generally used individually at a level of from about 0.01% to 10% by weight, based on the total composition, preferably from about 0.05 to 5% by weight. In the aggregate, the total of such optional ingredients should not exceed about 20% by weight, based on the total composition, preferably not more than 15% by weight.

### EXAMPLES

### Example 1

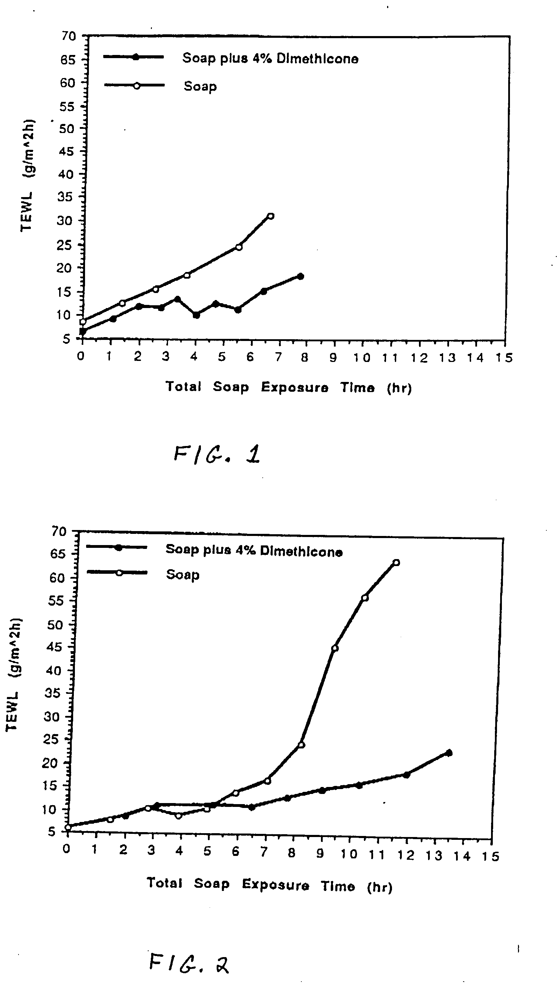

Reduction of surfactant damage to the skin's water barrier by water-insoluble silicone is evaluated in vitro by transepidermal water loss (TEWL). Experiments were conducted on a Servo-Med Evaporimeter at low relative humidity with a 60,000 centipoise dimethicone (polydimethylsiloxane) in a 60T/40C super fatted soap base and in a 70T/30PKO soap base, each containing 2% TiO₂. Repeated exposure of excised pig skin to 4% soap solutions without the dimethicone dramatically increases evaporative water loss, which is indicative of damage to the skin's water barrier. Soap solutions (4%) in water (33°C) made from bars containing 4% dimethicone prevent this increase in evaporative water loss observed with soap alone. The results for the 60T/40C super fatted soap and the 70T/30PKO soap are set forth in Figs. 1 and 2, respectively.

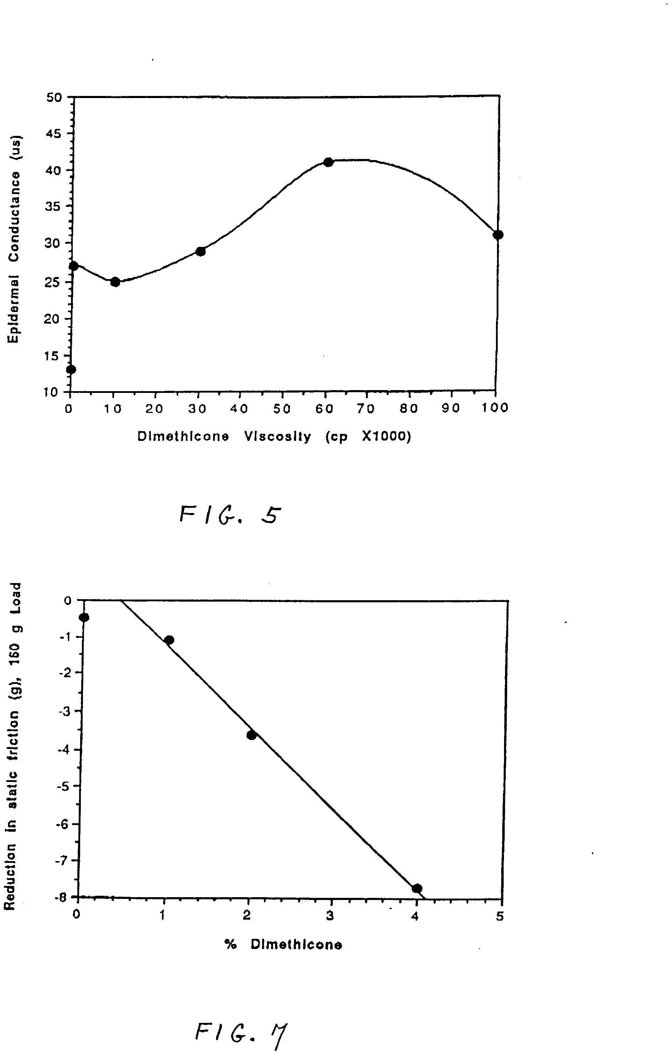

In order to evaluate the effect of silicone viscosity on surfactant (soap) damage to the skin's water barrier, the above procedure was repeated for compositions based on soap alone, or soap containing 4% dimethicone of 10,000 cP, 30,000 cP or 60,000 cP. The results are shown in Figure 6 where the TEWL values are normalized, i.e. are reported as a ratio of (TEWL after treatment)/(TEWL of untreated skin).

As can be seen from Fig. 6, there is substantially no difference between soap alone and soap plus dimethicone having a viscosity of 10,000 cP. The 60,000 cP dimethicone provides somewhat better protection against damage to the skin's water barrier than the 30,000 cP dimethicone after prolonged exposure (approximately 5 hours), however, both are significantly better than soap alone and soap plus 10,000 cP dimethicone.

### Example 2

Soap damage to the skin is measured for a range of dimethicone viscosities between 350 and 100,000 cP at identical weight concentrations of dimethicone when delivered from soap bars. The moisture retention (% area of dermal hydration) of excised pig skin is evaluated one hour after exposure to 4% soap (70T/30PKO) solutions made from bars containing 4% dimethicone. The results are set forth in Fig. 3.

The actual surface deposition of the silicone (reported as Si/N ratio) is measured using ESCA. The ESCA measurements, as set forth in Fig. 4, confirm a linear increase in dimethicone deposition with increasing polymer viscosity.

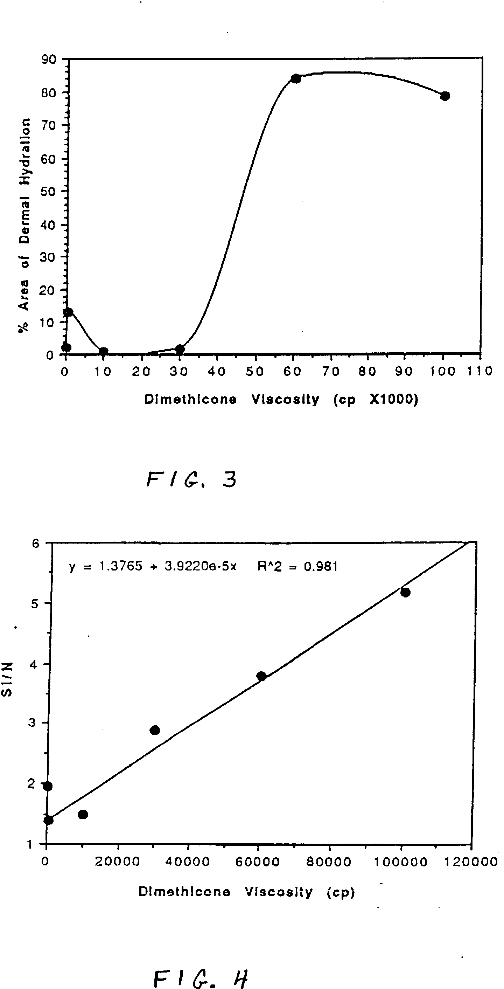

Additionally, as shown in Fig. 5, skin conductance, a measure of moisturization, also increases with increasing dimethicone viscosity, reaching a maximum at approximately 60,000 cP.

### Example 3

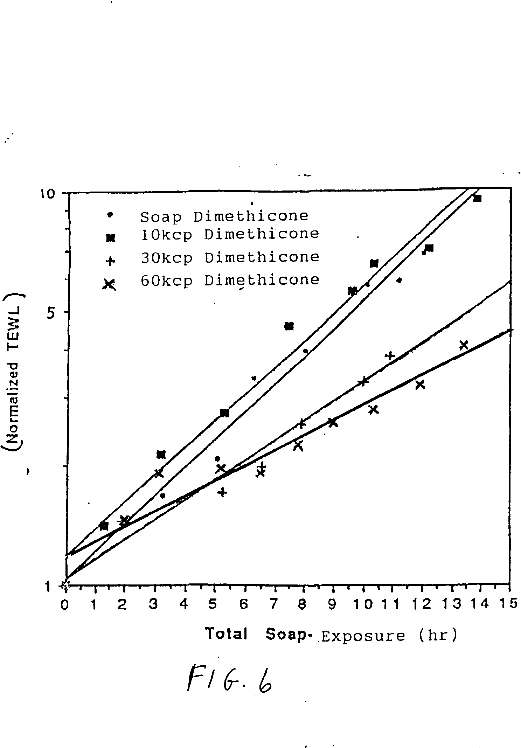

Skin friction is measured for skin samples treated by soap alone and by soap plus dimethicone. Static friction decreases linearly with increasing dimethicone (60,000 cP) concentration delivered as a single application from a 70T/30PKO bar, in the same manner as set forth above, as shown in Fig. 7.

### Example 4

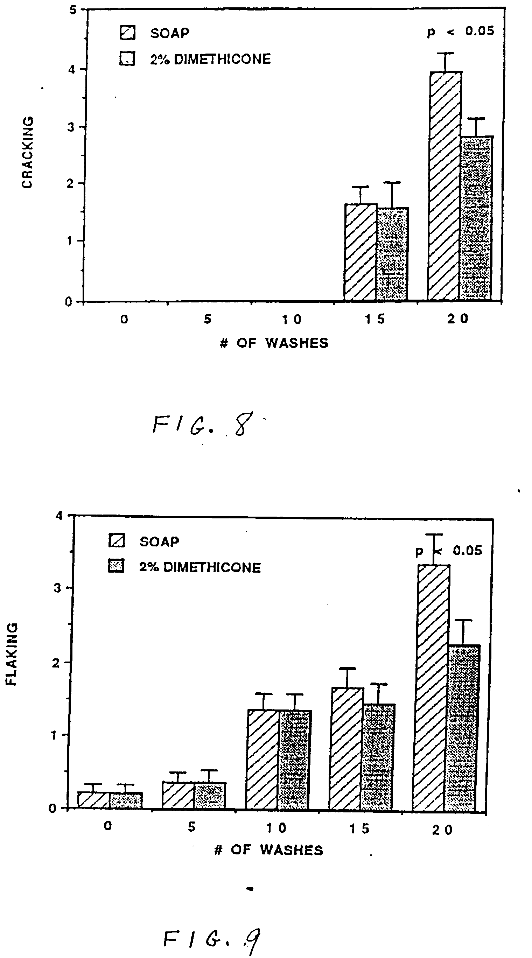

A panel test was performed to determine whether the protective effects observed with the 60,000 cP dimethicone, in vitro, occurred in vivo. The dimethicone was incorporated into a 70T/30PKO bar for the study. Soap induced cracking (Fig. 8) and flaking (Fig. 9) were reduced for the bar containing 2% dimethicone relative to the soap alone (p ≦ 0.05). In these tests, the panelists are asked to rate each soap bar composition on a scale of 0 to 6, where 0 represents no flaking or no cracking and 6 represents unacceptably high , excessive flaking or cracking.

No differences in erythema were observed. Edema was not produced during the study. Seven of the eleven panelists who finished the study stated that the dimethicone treated hand "felt better", while three were unable to distinguish between the two hands. Only one panelist chose the soap hand. Although not statistically significant, this sensory observation does directionally suggest that the subject could identify positive attributes of the dimethicone incorporation.

### Example 5

A larger panel test (N=29) was performed on a "half-face study" in which each panelist washed one-half of his or her face with a bar of soap alone and the other half with a bar of soap plus dimethicone (60,000 cP). The panelists were asked to evaluate the softness of each treated "half-face". After 5 minutes, the panelists reported that the dimethicone/soap treated half was statistically significantly softer (p ≦ 0.05) than the soap-only treated half. After 30 minutes, these panelists reported that the soap/dimethicone treated half was directionally significantly (p ≦ 0.1) softer than the soap-only treated half.

Within this test panel, 14 of the 29 panelists were trained panelists having previous experience in evaluation of soap bar or other cosmetic products. Among this sub-population (N=14), the following results were reported.

| TIME of APPLICATION | SOAP/DIMETHICONE vs. SOAP ALONE |
|---|---|
| 5 Minutes | . Significant increase in softness |
| | . Significant increase in moisture |
| | . Directional increase in smoothness |
| | . Directional increase in "feels dry" |
| 30 Minutes | . Significant increase in smoothness |
| | . Directional incease in softness |
| | . Directional increase in moisture |
| | . Directional increase in "feels dry" |

Polydispersity is the weight average molecular weight divided by the number average molecular weight.

## Claims

1. A solid soap composition for cleaning and conditioning hair and skin comprising:
from 93% to 99.5% by weight of a soap base; and
from 7% to 0.5% by weight of a water-insoluble silicone having a viscosity in the range of from 20,000 to 200,000 cP.

2. A solid soap composition as claimed in claim 1, characterised in that the viscosity of the water-insoluble silicone is in the range of from about 25,000 cP to 150,000 cP.

3. A solid soap composition as claimed in any preceding claim, characterised in that the viscosity of the water-insoluble silicone is in the range of from 30,000 cP to 100,000 cP.

4. A solid soap composition as claimed in any preceding claim characterised in that the number average molecular weight of the water-insoluble silicone is from 15,000 to 35,000.

5. A solid soap composition as claimed in any one of the preceding claims, characterised in that the said water-insoluble silicone is present in an amount of about 2 to about 4% by weight.

6. A solid soap composition as claimed in any one of the preceding claims, characterised in that the said water-insoluble silicone has a number average molecular weight in the range of from about 20,000 to about 30,000.

7. A solid soap composition as claimed in any one of the preceding claims, characterised in that the said water-insoluble silicone is a polydihydrocarbyl-siloxane.

8. A solid soap composition as claimed in claim 6, characterised in that the said polydihydrocarbylsiloxane is polydimethylsiloxane.

9. A solid soap composition as claimed in any one of the preceding claims, characterised in that the said soap base comprises a mixture of soaps.

10. A solid soap composition as claimed in claim 9, characterised in that the said mixture of soaps comprises a major portion of tallow soap and a minor portion of an additional soap.

11. A solid soap composition as claimed in claim 10, characterised in that the said tallow soap comprises about 60 to about 70% by weight of said mixture of soaps.

12. A solid soap composition as claimed in claim 11, characterised in that the said mixture of soaps is a 60 tallow/40 coco soap mixture.

13. A solid soap composition as claimed in claim 11, characterised in that the said mixture of soaps is a 70 tallow/30 palm kernel oil soap.

14. A solid soap composition as claimed in claimed in any one of the preceding claims, characterised in that the said composition is in the form of a bar.

15. A solid soap composition according to any preceding claim wherein the water-insoluble silicone has a polydispersity less than or equal to 4.5.
